# EUROPEAN PATENT SPECIFICATION

(11) **EP 4 338 095 B1**
(45) Date of publication and mention of the grant of the patent: **18.02.2026**
(21) Application number: 22731725.2
(22) Date of filing: 04.05.2022
(51) Int. Cl.: G06K 19/07, G04G 21/02

(54) **RFID SENSOR ARRANGEMENT AND METHOD FOR ITS MANUFACTURE**
RFID-SENSORANORDNUNG UND VERFAHREN ZU IHRER HERSTELLUNG
DISPOSITIF DE CAPTEUR-RFID ET PROCEDE POUR SA FABRICATION

(30) Priority: 11.05.2021 FI 20215560
(43) Date of publication of application: 20.03.2024
(73) Proprietor: Elcoflex OY, 90440 Kempele (FI)
(72) Inventor: TARVAINEN, Timo, 90410 Oulu (FI); PELTONIEMI, Timo, 90440 Kempele (FI)
(74) Representative: Berggren Oy
(86) International application number: PCT/FI2022/050296
(87) International publication number: WO 2022/238614

(56) References cited:
- WO-A1-2019/234304
- US-A1- 2013 162 405
- US-A1- 2017 052 160
- KANTAREDDY S N R ET AL: "UHF RFID tag IC power mode switching for wireless sensing of resistive and electrochemical transduction modalities", 2018 IEEE INTERNATIONAL CONFERENCE ON RFID (RFID), IEEE, 10 April 2018 (2018-04-10), pages 1 - 8, XP033356614, DOI: 10.1109/RFID.2018.8376201

## Description

### Object of the invention

The invention relates to a sensor solution, which is based on an electrochemical cell or a group of cells, and data is sent onward with a particular RFID-based solution. The application areas of the invention comprise, for example, monitoring of food cold chains, and medicinal remote measurement applications.

### Background of the invention

Several research institutes and companies have made efforts to develop smart measuring stickers (Smart Label) either based on a microprocessor-based sensor solution (digital), or based directly on a phenomenon that changes physically or chemically (analog). Several examples of both solution models can be found on the market. The concept is usually based on an embedded system built around a microprocessor, where in addition to temperature measuring, also data processing and the control of other I/O functions are placed on one chip. A large number of pads leads to a fairly large chip size, because the resolution used in antennas, typically at the most about 200 µm pitches, limits the miniaturization of the circuit. E.g. an NHS3100 chip by NXP has 24 pads and its size is 2.51 x 2.51 mm. It would be possible to manufacture the chip more inexpensively with a 4-5 times smaller dimension, but then the cost of the antenna substrate and bonding process would rise significantly. The use of silica-based electronics leads to an expensive assembly also because the solution requires a separate battery among others for data reading and storage. The microprocessor solutions can also be read either with UHF or HF signal energy, whereby a battery is not essential. For the above-mentioned reasons, a Smart Label solution has not been able to replace recyclable measuring modules, the price range of which is 10-30 euro/piece.

"Kantareddy", "UHF RFID tag IC power mode switching for wireless sensing of resistive and electrochemical transduction modalities", 2018 IEEE International Conference on RFID, 10 April 2018, discloses a concept of using the change in the state of an RFID tag IC from passive to battery-assisted passive (BAP) as an indicator for sensing. This concept is well suited for transduction mechanisms that produce charge, such as electrochemical reactions through a case example in glucose sensing.

US 2017/0052160 ("Olsson") discloses a printed gas sensor and digital expiry date thereof. Olsson's printed gas sensor, comprising a metalloporphyrin dye, has first and second modes, and a nanoporous carrier material comprising a plurality of particles measuring less than 5 µm. The particles have a plurality of pores, the pore size in the range 5-50 nm, wherein the metalloporphyrin dye is bound to the nanoporous carrier material. Olsson further discloses a method of preparing a gas sensing composition for detection of food status and a digital expiry date device system, which comprises a packaging material with inner and outer surfaces; a first transponder disposed on the inner surface and a second transponder disposed on the outer surface. A sensor portion is electrically connected to the first transponder for detecting food status and communicating the status to the second transponder. The sensor portion comprises a metalloporphyrin dye which configures an impedance change upon binding with a gaseous analyte, and a printed numerical array.

US 2013/0162405 ("Forster") discloses a radio frequency identification sensor assembly. In accordance with one embodiment of Forster, an RFID sensor assembly comprises an RFID chip, an antenna, a sensor, a substrate, and a laminated layer. The RFID chip is in electrical communication with the antenna and the sensor. The RFID chip, antenna, and sensor are secured to the substrate. The laminated layer is arranged as to create a generally airtight pouch between the substrate and the laminate layer. The laminated layer is further arranged to facilitate a post manufacturing method for exposing the contents of the pouch to the environment surrounding the RFID sensor assembly.

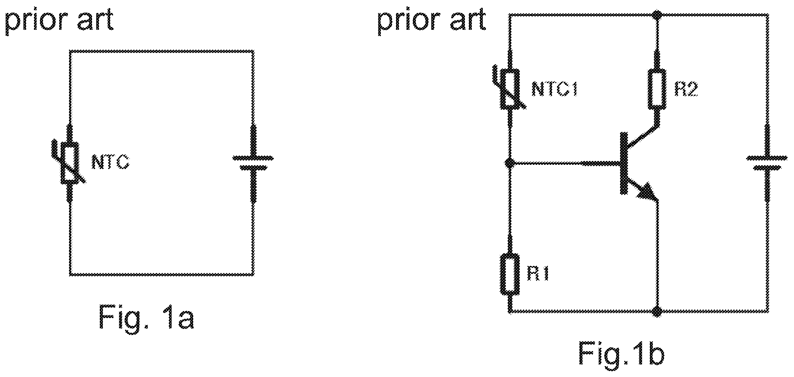

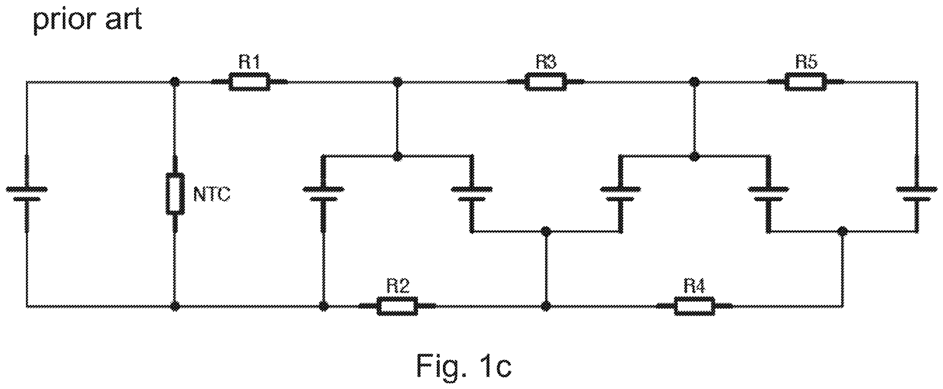

Elcoflex Oy's patent application FI 20185523 presents a TTI solution (Time-Temperature Indicator) based on electrochemical cells, where the charging state of the cell is interpreted from a change in the colour of an electrode. When the cell is discharged with the aid of an NTC resistor, the discharge situation is made dependent on, in addition to time, also temperature. The basic circuit is either just an NTC resistor and a cell (as in figure 1a, which is from application FI 20185523), or it additionally has a transistor, when it is desired to have a more precise coupling point for temperature (figure 1b, also from application FI 20185523). By coupling the cells consecutively, so that they discharge sequentially (i.e. one after the other), a better resolution is obtained for optically readable cells. There is an example of this in figure 1c, which has an NTC resistor, six cells and suitable discharge resistors in the shown coupling.

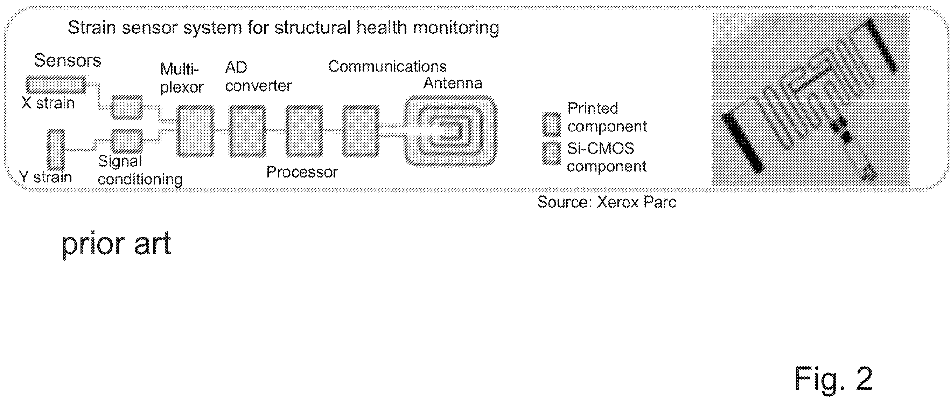

By using a microprocessor and a temperature sensor, the TTI in question can be manufactured, which is also able to store the measured temperatures. Such a circuit is e.g. NHS3100 by NXP, which contains a temperature sensor, and the data of which can be read with an NFC connection. The solution is, however, expensive and therefore cannot become widespread in volume products. The general solution model (figure 2) presented by Xerox describes a sensor arrangement for monitoring health data. In this solution, there are strain gauges in the X and Y direction, the signal data measured by which is processed, multiplexed, AD converted to processor input data. The data is sent onward via a communication output to an antenna. In this Xerox structure, the processor block, AD converter and output circuit toward the antenna are manufactured with silica-based Si-CMOS components. On the other hand, the sensors, the pre-processing circuits for the signals measured by them, the multiplexer and the antenna could be implemented as printed components or parts. In this model according to figure 2, the whole crux of the problem is summarized: When a microprocessor that is over a million times faster is used for processing analog signals based on minutes time constants in the digital domain, the function of which microprocessor is designed for communication on RFID frequencies, we drift into an overall ineffective and expensive solution. The main part of the system effect is underutilized. Thus a solution model, which is developed for analysing rapidly changing signals, is not, due to the cost structure, suitable for example for measuring the freshness of food products, where the time constant is minutes to hours. Transferring functions to the chip leads to pad limited design. The display control particularly requires a significant amount of silica area, which is often a more expensive part of the implementation than the price of the control logics of the display pixel itself. The Acreo/RISE research institute has recognized this problem and demonstrated a printed display controller with organic transistors.

The prior art thus has a clear problem area, which the present invention sets out to solve.

### Brief summary of the invention

The present invention presents a device in accordance with claim 1.

In one embodiment of the invention, one or more cells of a cell group have been connected to the input of the RFID chip (32, 41, 52, 59), and a change in the voltage of said connected cells is arranged to be stored in the memory of the RFID chip (32, 41, 52, 59).

In one embodiment of the invention, the second-to-last cell (45) of the cell group (42, 43, 45) is arranged in the tamper input to indicate that the "best before" time limit of a food product or other product has passed, and the last cell (42) of the cell group (42, 43, 45) is arranged in the tamper input to indicate that the "last day of use" time limit of a food product or other product has passed.

In one embodiment of the invention, the emptying of a desired cell in the cell group (42, 43, 45) causes the RFID chip's (32, 41, 52, 59) tamper input in question to turn to be logically opposite.

In one embodiment of the invention, the device has an NFC (46), LF, Bluetooth or UHF antenna for sending data onward.

In one embodiment of the invention, the cells of the cell group comprised in the device, the ohmic resistors (R1-R8) and the antenna (50) are manufactured by printing with a roll-to-roll technique, and the NTC resistor (51) and transistors comprised in the device are implemented with discrete circuit components with soldering or glue technique.

In one embodiment of the invention, the output voltage of the cell connected to the input is a function of some measurable physical quantity or quantities, and the measuring time.

In one embodiment of the invention, said physical quantity is temperature, relative humidity, electromagnetic radiation, and/or vibration.

In one embodiment of the invention, the device is arranged to discharge or charge the charge of the cell with a piezo- or magnetoresistive component.

In one embodiment of the invention, the discharge time of the cell or cell group has been set to be determined based on static pressure or impacts by using electroactive plastics.

In one embodiment of the invention, the first cell (43) of the cell group is placed outside the RFID tag containing the RFID chip (32, 41, 52, 59) and the electric circuit of said first cell (43) is cut when the device is taken into use.

In one embodiment of the invention, the circuit connected to the input of the RFID chip (32, 41, 52, 59) is made up of an analog computer (31) comprising a transistor or transistors.

In one embodiment of the invention, the reading device is arranged to receive wireless data sent by the RFID tag with NFC technology or Bluetooth technology or an UHF antenna or an LF antenna.

In one embodiment of the invention, the device is arranged to be applied to the monitoring of the cold chain of food products, the monitoring of humidity exposure of products, quality control of high end type products, the monitoring of temperatures required by medical products such as vaccines and medicines, biomedical (remote) measurements, or measuring and monitoring of physical product damages in logistics.

### Description of the drawings

Figure 1a shows an electrochemical cell solution in prior art, which has an NTC resistor and a cell,
Figure 1b shows an electrochemical cell solution in prior art, which has an NTC resistor, a cell, and a transistor with a suitable resistor coupling,
Figure 1c shows an electrochemical solution with successive cells in prior art, which has an NTC resistor, six cells and suitable discharge resistors,
Figure 2 shows a common solution model presented by Xerox, a so-called IdTechEx, with its physical parts related to health monitoring,
Figure 3 shows a flowchart of a solution according to the general form of the invention for measuring any quantity dependent on time and for relaying the data in a wireless manner with the aid of an RFID chip and an antenna,
Figure 4 shows an example of a TTI functionality by using the tamper input of the RFID chip by connecting the last cell thereto with a reverse polarity,
Figure 5a shows an example of a conceptual circuit diagram, which has an NTC resistor, six transistors, and one cell with necessary resistors, and an RFID chip with antenna, and
Figure 5b shows an example of an NFC tag with a 15 x 15 mm size, which includes a temperature sensor.

### Detailed description of the invention

The present invention presents a new solution for the above-described problem, where the solution is also advantageous with regards to manufacturing technique (i.e. inexpensive), suitable for large volumes and where optical interpretation (i.e. manually done by a user) is no longer required for the colour change indicated by the electrochemical indicator. The presented solution also makes possible data transfer over a longer distance using RFID technology, and this in turn makes possible several different applications in the field of food monitoring and medical applications. For example food administrations (such as the FDA in the USA) typically require that product monitoring also involves obtaining identification data of the product, i.e. precise individualization of the product.

The only way to scale TTI to large volumes is to either read the result from a printed circuit functioning in a completely analog manner, e.g. optically with the aid of a QR code, or to convert the result calculated in an analog manner to a digital one and use an inexpensive RFID chip for the reading. Analog solutions have been developed by e.g. Timestrip (https://timestrip.com/products/food-range/) and Insignia Technologies, but they lack the product identification. Infratab has designed an UHF/NFC freshness sticker based on a microprocessor, but its price is very high. In order to lower costs, commercial RFID chips must be used, the volumes of which are large and the size of the chip small. A typical RFID chip size is 0.5 x 0.5 mm and its manufacturing and tag assembly technology is mature due to large manufacturing volumes, which has been 20 billion pieces in 2020. There are currently NFC and UHF chips on the market, which have a so-called tamper function, which is based on a breaking resistive circuit, i.e. a so-called tamper loop. The tamper data is stored in the chip memory and cannot be changed afterwards. The operation of the chip is possible to implement also in the analog circuit of the previously presented Elcoflex patent application (FI 20185523), so that the voltage of the last cell in the circuit is used to control the tamper function of the digital system. This is one embodiment of the present invention. In this case, the cell does not need to be optically readable, which is the biggest difference compared to patent application publication FI 20185523. The solution in question according to the invention uses analog data processing for converting the exceeding of a limit value of a slowly changing quantity into a digitally readable and storable form. Because the analog components can be printed on R2R lines (roll-to-roll) and they are based on inexpensive materials, and on the other hand RFID chips are manufactured in large volumes, it is possible to attain an inexpensive structure, which is additionally scalable to large volumes. A cost estimate for the above-mentioned sticker at this time is 10-20 cents, which is a fraction of the cost of a microprocessor-based solution. The solution presented by the invention does not require changes to the infrastructure as such, but all existing reading devices and cloud services designed for tamper function are valid. It is to be expected that in the future, the functionality of the RFID chip grows, even though the dimensions do not grow, and more Analog Computer calculation results can be indicated e.g. with PWM (pulse-width modulation) or other modulation technique without increasing the size of the chip.

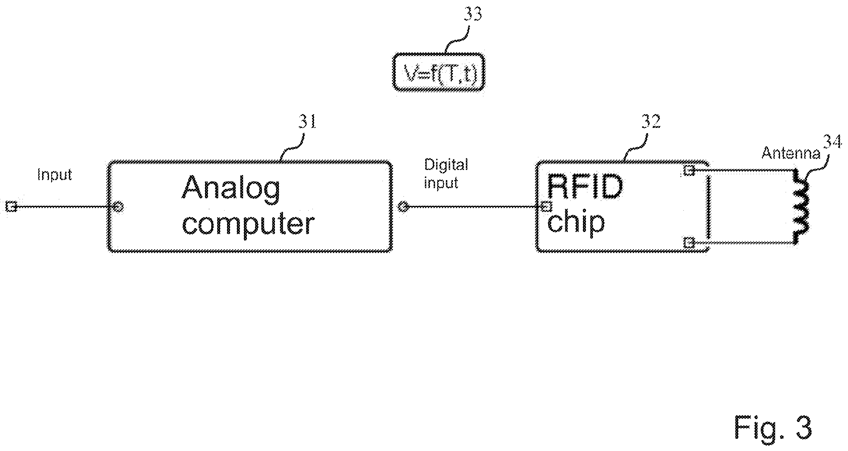

Figure 3 shows a flowchart of a general solution for measuring any quantity dependent on time and for relaying the data in a wireless manner with the aid of an RFID chip 32 and an antenna 34. This is applied in one example of the invention. The Analog Computer 31 seen in the left half of figure 3 processes the measured quantity based on a predetermined function and converts the data to be suitable for the digital input of the RFID chip 32. The output voltage 33 is a function of time and temperature, i.e. V=f(T,t), or of some other measurable quantity or quantities. When the output voltage of the analog circuit reaches a predetermined value (as the cell empties), the digital input of the RFID chip 32 converts it to logical ON/OFF data, which is communicated wirelessly to the RFID tag reading device via the antenna 34. The inventiveness of the presented solution is in its way to use an electrochemical cell in the RFID chip 32 interface as a kind of one-bit analog-digital converter and simultaneously replace the real-time clock implemented with a microprocessor. The battery is thus used as a clock as one would use an hourglass. Using an RFID chip 32 also enables the possibility for identifying and tracing the product, which is required e.g. in the FDA's (US Food and Drug Administration) document: "New Era of Smarter Food Safety - FDA's Blueprint for the Future". There, the required issues are phrased like this in two essential points:
"Encourage the use of technology that automatically monitors product risk factors associated with new business models, such as time, temperature, tamper resistance, and traceability information.
Encourage food traceability technology providers to develop creative financial models that are low to no-cost solutions, proportional to benefits derived from participating, and enabling food producers of all sizes to participate in a scalable, cost-effective way."

The present invention fulfills, among others, these requirements well.

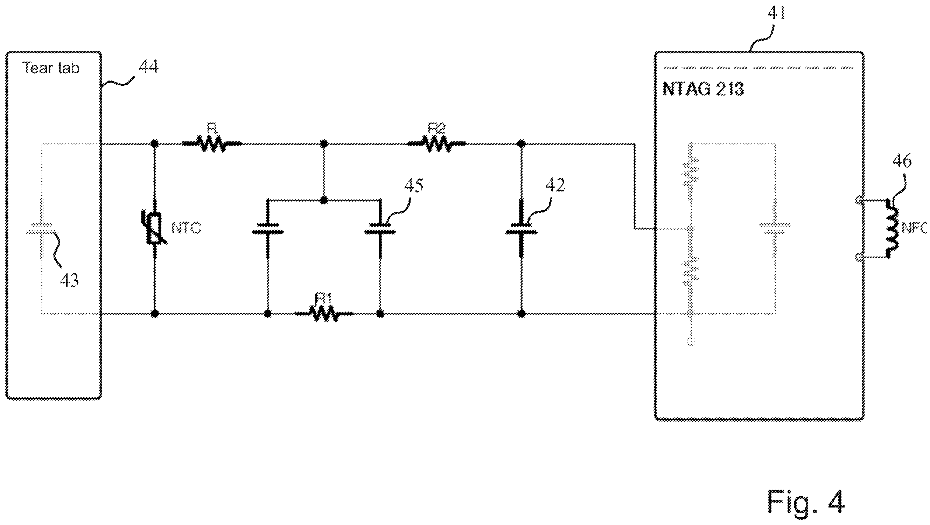

Figure 4 in turn shows a TTI functionality by using the tamper input of the NTAG 213-RFID chip 41 by connecting the last cell 42 thereto with a reverse polarity. This represents one embodiment of the invention. As long as the cell 42 has a charge, the current caused thereby to the tamper input keeps the voltage below a determined limit value. When the charge of the cell 42 has been used up, the internal connection of the NTAG 213-RFID chip 41 causes the tamper input to move to the logical 1 state, which corresponds to a situation, where the tamper loop according to the original purpose was broken. Thus, discharging of the charge effectively corresponds to breaking of the tamper loop, which data is stored in the memory of the RFID chip 41. In this context, "tamper" can be interpreted broadly to mean spoiled, exposed, frozen or otherwise ruined, to name a few examples. In the coupling in figure 4, all the components, with the exception of the chip 41, are printed and thus very inexpensive to manufacture. This represents a useful embodiment of the invention with regards to cost. When the first cell 43 is placed outside the RFID tag, so that its electric circuit breaks when the tag is taken into use, an automatically starting sticker is achieved, the storage time of which can be adjusted with the capacity of the external cell. This functionality is thus seen in figure 4 in the form of a tear tab 44, where in connection with the tearing of the tab 44, the connection of the first cell 43 to the rest of the electric circuit is electrically broken. As an alternative, a printed transistor can be used like a switch, which switches on the cell or cell group, when the gate voltage of the transistor is cut in one embodiment of the invention.

The RFID chip 41 output has an antenna 46 supporting e.g. NFC technology. Alternatively, the output can have an LF, Bluetooth or UHF antenna.

RFID means identification based on wireless communication. Thus, an identifier operating e.g. on a Bluetooth frequency is an RFID, when it contains an individual code. One possible new type of product could be a Bluetooth chip based on energy harvesting, which has, in addition to an individualized code, also one or more inputs for identifying physical variables. The data can be sent over a distance of more than 100 meters with the power of collected energy. This is one example of the invention.

In general, four types of RFID identifiers are commonly used nowadays, which differ from each other based on the used radio frequency: low frequency (LF) identifiers (125-134 kHz / Wikipedia 2016; 120-150 kHz / Wikipedia 05/2021), high frequency (HF) identifiers (13.56 MHz), UHF-identifiers (868-956 MHz / Wikipedia 2016; 865-868 MHz (Europe, Wikipedia 05/2021) or 902-928 MHz (North America, Wikipedia 05/2021)) and microwave identifiers (2.45 GHz; and also 5.8 GHz; Wikipedia 05/2021). Of these, only the frequency bands 2.45 GHz and 5.8 GHz are available internationally, because they are in the freely usable ISM frequency range (ISM = "Industrial, Scientific, and Medical purposes").

The accuracy of the time x temperature input is determined by the weight accuracy of the resistors and the cell electrodes. In the coupling of figure 4 it is also possible to, in addition to the data on the RFID chip 41, use as additional data optical interpretation according to our earlier patent application FI 20185523 for example when it is desired to more precisely clarify the status of the data stored on the chip 41. For example, when the second-to-last cell 45 in figure 4 is connected to the input of the RFID circuit, the emptying of the cell in question indicates that the "best before" state has passed and the last cell 42 can further indicate if the product is suitable for use. When the NFC chip is replaced by an UHF chip, it is possible to read the TT data from a distance of up to 10 meters. The solution makes possible for example automatically remotely readable food freshness stickers and recognizes products that were spoiled during transport. The coupling of electrochemical cells in question can be used in all resistively readable sensors and to convert data to be remotely readable with the aid of a discharging and in one coupling version also charging cell. By using an electrochemical cell in addition to the resistive sensor, the time function can be obtained in the measuring without expensive electronics. By adding printed electronics such as transistors, condensers and coils to the measuring circuit, an analog circuit can be formed, with the aid of which the cell can be discharged with something other than a resistive sensor, and the tamper input can be utilized for communicating data calculated in an analog manner.

In other words, in the described solution, the electrochemical cell is used as a component in an analog data collecting arrangement to replace digital time calculation, processing and memory. Because the coupling does not require a separate battery, the cell makes it possible to process information from the analog sensor with a small power consumption and to calculate the final result as a combination of the sensor data, time and cell capacity. The cell is thus not the power source of the RFID chip connected thereto, but the RFID chip obtains its operating voltage from the RFID field. When the charging of the cell is discharged, it simultaneously functions as a memory, even though the RFID chip is not in operation. When the RFID chip is read, the data is only at that time stored in the RFID chip memory. It is appropriate to stress that even though the temperature is the most common quantity to be measured, in its stead can just as well be humidity, and the output voltage of the analog circuit is thus a function of the relative humidity and time, i.e. V=f(RH,t) (RH = relative humidity), and the calculated result can be a combination of several variables.

One advantageous embodiment is a conductive polymer semiconductor based (conjugated polymer) ion gated OFET, i.e. organic electrochemical transistor (OECT). Its slowness is not a problem in this application and the small operating voltage can be implemented advantageously with a zinc-manganese cell, which is recyclable. Mass manufacturing is possible with an R2R screen printing apparatus in normal atmosphere.

In one embodiment of the invention, a charging cell can be used instead of a discharging cell. Thus, it is possible to construct a system equipped with a battery, where the battery is meant as a source of operating voltage and the time-axis of the analog calculation is implemented by a second chargeable and dischargeable cell. When the charge of the cell exceeds a certain level (i.e. a limit value), this level is indicated in the RFID chip input. The solution is structurally more expensive than a structure applying a discharging cell. Another use purpose of a chargeable cell could be its function as an optical indicator in accordance with Elcoflex's previous patent application FI 20185523. In this case, in accordance with what is described above, the last cell implements an "hourglass" function, but instead of discharging the battery charge with a resistor, it is used for indicating intermediate stages of the analog calculation. This would be useful when the RFID tag of the product informs that the product has been spoiled and there is a desire to know, how far the process has advanced. Thus, the optical data has additional value. In this case, the optical cell can be any electrochemical cell, e.g. electrochromatic.

With reference to the above-mentioned "best before" and "product still usable" states in connection with the description of the data of the last two cells, the tag in other words indicates that the product has already slightly expired, but if the last cell has not yet discharged (or charged), the product could still have sales value. There could thus be a simple reader at the check-out, with which the charge of the cell in question could be identified e.g. inductively or optically, in one example of the invention.

In the following, some application areas of the invention will be discussed.

One of the application fields with the most potential is Cold Chain management for food and also for medical field products such as vaccines. When an organic transistor is used in the circuit in addition to the NTC resistor for modifying the graph of the NTC resistor, so that the resistance decreases sharply above +3 °C, an effective indicator is obtained for example for fish or meat packages to prevent botulism.

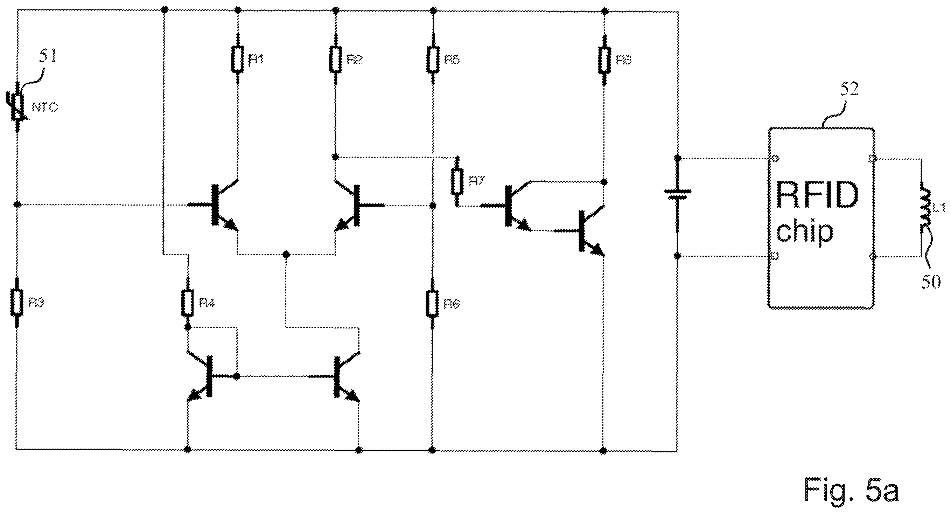

Figure 5a shows an equivalent coupling for one possible solution of the invention with bipolar transistors, of which there are six in this example. The estimated printed area of the chip is less than 1 cm². The circuit functions so that when a thermistor 51 has reached a temperature limit set with a resistor chain R5 and R6, a differential amplifier Q1/Q2 (the two transistors in the middle of the diagram) controls the output stage Q5/Q6 (the two transistors on the right in the diagram) to be switched on and the resistor R8 empties the battery (for example a 1.5 volt cell) in a predetermined time, which in turn switches the tamper bit of the RFID chip 52 to one. The RFID chip 52 output has an antenna 50. The coupling is a principled concept and it requires changes, when the coupling is implemented with an OECT. With current OECT transistor models, the enclosed coupling can be printed in connection with the antenna on a space of under 10 x 10 mm and a smart label can be manufactured at a price of about 10 cents. Because the size of the antenna affects the reading distance, it can not be very miniaturized. If the transistor can be manufactured with screen printing technique on an 0.5 x 0.5. mm area, it would be possible to get 1600 transistors in total on a 10 x 10 mm area. This requires the printing of a second conductor layer for the couplings between transistors, but this does not add to the costs of NFC tags, because the second layer is in use either way when forming a coil. Here is one advantage of the structure in question.

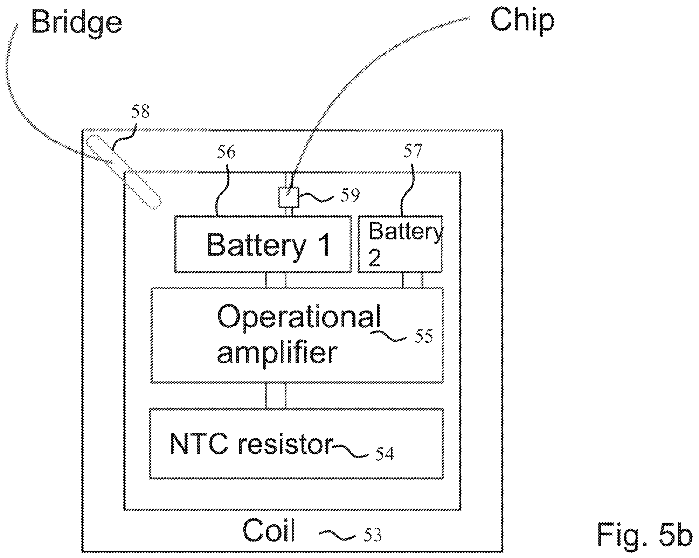

Figure 5b in turn has an example of 15 x 15 mm size NFC tag, which has a temperature sensor, i.e. an NTC resistor 54. This figure shows the placement of components on the area of a typical NFC antenna in one example of the invention. On the circumference there is a copper etched coil 53, one end of which is connected to the antenna outlet of the chip 59 with the aid of a printed bridge 58. An operational amplifier 55 and the NTC resistor 54 are printed components, which discharge the charge of "battery 1" (i.e. Batt_1) 56. The "battery 2" (i.e. B_2) 57 creates a reverse gate voltage when a p-channel OECT is used. Because the gate voltage is very low, the size of this battery is also small. "Battery 1" 56 is connected to the input of the chip 59.

Even though the smart label according to the coupling above (figure 5a) is best suited for R2R manufacturing, which attains large volumes and an inexpensive price level, the circuit can in one example of the invention be manufactured also with discrete components either on an FR4 or flex base. Thus, the antenna, resistors and battery are manufactured by printing, and the bipolar transistors and NTC resistor are stacked either with soldering technique or glue technique. The solution leads to a more expensive price, but the accuracy is better than with printed components. E.g. with BC847BV dual SOT-666 transistors and a stacked 0402 NTC resistor (TX04F103F3380ER) the price rises by 10-15 cents, which would still be only a fraction of the price of an embedded system. Due to the higher price and better accuracy, the product would be suited for monitoring high-end products.

For moisture sensitive products, the monitoring can be done in a corresponding manner using a printed humidity sensor. By adding components to the analog part, a measuring can be implemented, where V=f(RH,T,t) when the spoiling of the product is affected by, in addition to temperature, also humidity and/or radiation. In the medical field, the NTC resistor graph can be tuned e.g. to the temperature range required by a vaccine. When the expiry mechanism of the product is almost linear (time x temperature), the result can at its simplest be obtained with only an NTC resistor and a cell, especially when the phenomenon to be measured is based on the Arrhenius law and the exponential R=A exp(B/T) graph of the NTC resistor is directly applicable. It is also known practice that the graph of the NTC resistor can be modified with resistor networks to correspond to the characteristics of the physical phenomenon being studied. Mechanical vibration, heat conduction, UV, IR or other electromagnetic radiation can be measured in a corresponding manner by discharging the charge of the cell with a component reacting to the physical quantity in question. The component can also be a piezo- or magnetoresistive component, which the aid of which the cell is discharged or charged. By using electroactive plastics (Arkema), it is possible to make an analog coupling, where the discharge time of the cell is determined based on static pressure or impacts. The use of such a sensor in the above-mentioned way connected to an RFID chip makes possible inexpensive damage monitoring in logistics. Current sensors are usually based on a relatively large mass (F=m*a), so an analog printed coupling probably requires reinforcement (i.e. a transistor), so that the masses can be kept small and the products can be printed with R2R technique.

One potential growth area is biometric analyzing and for example combining monitoring results of patients in the above-mentioned manner into an RFID-readable circuit. An analog part connected to the RFID chip, possibly containing biotransistors, which measure alterations in vital functions, would be an advantageous way to monitor a patient and direct him/her to further care after an alarm has occurred. The reading device could in the NFC case be a mobile device, whereby the monitoring would be done on one's own initiative. When UHF technology is used, the reading could occur automatically, without actions by the patient. With the aid of this technology, significant savings can be obtained in patent monitoring. Even though the RFID technology is ready for taking the application into use, printed biometric reliable sensors are just being developed.

In this context we want to stress the inexpensiveness of the solution of the invention and its compatibility with current RFID infrastructure compared to other monitoring products on the market. Because the product is based on existing manufacturing technology, it can be scaled to current antenna production volumes, which is 1-10 millions/production batch. Scalability in manufacturing is also a requirement for the technology becoming widespread. Because the price level remains at a level of 5-10 cents, it cannot yet be applied to all food products, such as for example dairy products, but it could become more common in packages costing several euros and in medical field products. If the manufacturing of the transistor can be implemented in the future with engraving printing technique combined with AI-UHF antennas, the price could fall close to the current level of an UHF antenna.

The invention is not limited only to the embodiments presented above, but the invention can vary within the protective scope defined by the enclosed claims.

## Claims

1. A device for indicating at least one physical quantity and/or the passage of time and for sending it electrically, wherein the device comprises a battery-less RFID chip (32, 41, 52, 59), and an electrochemical cell or cell group (42, 43, 45) connected to the input of the RFID chip, where the discharging or charging or a change in the charging state of the last cell of the cell or cell group (42, 43, 45) causes the input of the RFID chip (32, 41, 52, 59) to turn to be logically opposite, where the device includes RFID identification data.

2. The device according to claim 1, **characterized in that** one or more cells of a cell group have been connected to the input of the RFID chip (32, 41, 52, 59), and a change in the voltage of said connected cells is arranged to be stored in the memory of the RFID chip (32, 41, 52, 59).

3. The device according to claim 2, **characterized in that** the second-to-last cell (45) of the cell group (42, 43, 45) is arranged in the input of the RFID chip (32, 41, 52, 59) to indicate that the "best before" time limit of a food product or other product has passed, and the last cell (42) of the cell group (42, 43, 45) is arranged in the input of the RFID chip (32, 41, 52, 59) to indicate the "last day of use" time limit of a food product or other product has passed.

4. The device according to any of the claims 1-3, **characterized in that** the emptying of a desired cell in the cell group (42, 43, 45) causes the input of the RFID chip (32, 41, 52, 59) in question to turn to be logically opposite.

5. The device according to any of the claims 1-4, **characterized in that** the device has an NFC (46), LF, Bluetooth or UHF antenna for sending data onward.

6. The device according to any of the claims 1-5, **characterized in that** the cells of the cell groups comprised in the device, the ohmic resistors (R1-R8) and the antenna (50) are manufactured by printing with a roll-to-roll technique, and the NTC resistor (51) and transistors comprised in the device are implemented with discrete circuit components with soldering or glue technique.

7. The device according to any of the claims 1-6, **characterized in that** the output voltage of the cell connected to the input is a function of some measurable physical quantity or quantities and the measuring time.

8. The device according to claim 7, **characterized in that** said physical quantity is temperature, relative humidity, electromagnetic radiation and/or vibration.

9. The device according to any of the claims 1-8, **characterized in that** the device is arranged to discharge or charge the charge of the cell with a piezo- or magnetoresistive component.

10. The device according to any of the claims 1-9, **characterized in that** the discharge time of the cell or cell group has been set to be determined based on static pressure or impacts by using electroactive plastics.

11. The device according to any of the claims 1-10, **characterized in that** the first cell (43) of the cell group is placed outside an RFID tag containing the RFID chip (32, 41, 52, 59) and the electric circuit of said first cell (43) is cut when the device is taken into use.

12. The device according to any of the claims 1-11, **characterized in that** the circuit connected to the input of the RFID chip (32, 41, 52, 59) is made up of an analog computer (31) comprising a transistor or transistors.

13. The device according to any of the claims 1-12, **characterized in that** the device is arranged to be applied to the monitoring of the cold chain of food products, the monitoring of humidity exposure of products, quality control of products, the monitoring of temperatures required by medical products such as vaccines and medicines, biomedical (remote) measurements or measuring and monitoring of physical product damages in logistics.

14. A system for indicating at least one physical quantity and/or the passage of time and for sending it electrically, the system comprising the device according to any of the claims 1-12, **characterized in that** a reading device is arranged to receive wireless data sent by an RFID tag with NFC technology or Bluetooth technology or an UHF antenna or an LF antenna.

## Patentansprüche

1. Vorrichtung zur Angabe mindestens einer physikalischen Größe und/oder des Zeitablaufs und zu deren elektrischer Übertragung, wobei die Vorrichtung einen batterielosen RFID-Chip (32, 41, 52, 59) und eine elektrochemische Zelle oder Zellgruppe (42, 43, 45) umfasst, die mit dem Eingang des RFID-Chips verbunden ist, wobei das Entladen oder Laden oder eine Änderung des Ladezustands der letzten Zelle der Zelle oder Zellgruppe (42, 43, 45) bewirkt, dass der Eingang des RFID-Chips (32, 41, 52, 59) logisch entgegengesetzt wird, wobei die Vorrichtung RFID-Identifikationsdaten einschließt.

2. Vorrichtung nach Anspruch 1, **dadurch gekennzeichnet, dass** eine oder mehrere Zellen einer Zellengruppe mit dem Eingang des RFID-Chips (32, 41, 52, 59) verbunden sind und eine Änderung der Spannung der verbundenen Zellen so angeordnet ist, dass sie im Speicher des RFID-Chips (32, 41, 52, 59) gespeichert wird.

3. Vorrichtung nach Anspruch 2, **dadurch gekennzeichnet, dass** die vorletzte Zelle (45) der Zellgruppe (42, 43, 45) im Eingang des RFID-Chips (32, 41, 52, 59) angeordnet ist, um anzugeben, dass die Mindesthaltbarkeitsfrist eines Lebensmittels oder eines anderen Produkts abgelaufen ist, und die letzte Zelle (42) der Zellgruppe (42, 43, 45) im Eingang des RFID-Chips (32, 41, 52, 59) angeordnet ist, um anzugeben, dass die Mindesthaltbarkeitsfrist eines Lebensmittelprodukts oder eines anderen Produkts abgelaufen ist.

4. Vorrichtung nach einem der Ansprüche 1 bis 3, **dadurch gekennzeichnet, dass** das Entladen einer gewünschten Zelle in der Zellengruppe (42, 43, 45) dazu führt, dass der Eingang des betreffenden RFID-Chips (32, 41, 52, 59) sich logisch ins Gegenteil verkehrt.

5. Vorrichtung nach einem der Ansprüche 1 bis 4, **dadurch gekennzeichnet, dass** die Vorrichtung eine NFC-(46), LF-, Bluetooth- oder UHF-Antenne zum Weiterleiten von Daten aufweist.

6. Vorrichtung nach einem der Ansprüche 1 bis 5, **dadurch gekennzeichnet, dass** die Zellen der in der Vorrichtung enthaltenen Zellgruppen, die ohmschen Widerstände (R1-R8) und die Antenne (50) durch Bedrucken mit einer Rolle-zu-Rolle-Technik hergestellt werden und der NTC-Widerstand (51) und die in der Vorrichtung enthaltenen Transistoren mit diskreten Schaltungskomponenten mit Löt- oder Klebstofftechnik implementiert werden.

7. Vorrichtung nach einem der Ansprüche 1 bis 6, **dadurch gekennzeichnet, dass** die Ausgangsspannung der mit dem Eingang verbundenen Zelle eine Funktion einer oder mehrerer messbarer physikalischer Größen und der Messzeit ist.

8. Vorrichtung nach Anspruch 7, **dadurch gekennzeichnet, dass** die physikalische Größe Temperatur, relative Feuchtigkeit, elektromagnetische Strahlung und/oder Vibration ist.

9. Vorrichtung nach einem der Ansprüche 1 bis 8, **dadurch gekennzeichnet, dass** die Vorrichtung so eingerichtet ist, dass sie die Ladung der Zelle mit einer piezo- oder magnetoresistiven Komponente ablässt oder lädt.

10. Vorrichtung nach einem der Ansprüche 1 bis 9, **dadurch gekennzeichnet, dass** die Entladezeit der Zelle oder Zellgruppe basierend auf statischem Druck oder Einschlägen unter Verwendung elektroaktiver Kunststoffe bestimmt worden ist.

11. Vorrichtung nach einem der Ansprüche 1 bis 10, **dadurch gekennzeichnet, dass** die erste Zelle (43) der Zellengruppe außerhalb eines RFID-Tags angeordnet ist, das den RFID-Chip (32, 41, 52, 59) enthält, und die elektrische Schaltung der ersten Zelle (43) unterbrochen wird, wenn die Vorrichtung in Gebrauch genommen wird.

12. Vorrichtung nach einem der Ansprüche 1 bis 11, **dadurch gekennzeichnet, dass** die mit dem Eingang des RFID-Chips (32, 41, 52, 59) verbundene Schaltung aus einem Analogcomputer (31) besteht, der einen Transistor oder Transistoren umfasst.

13. Vorrichtung nach einem der Ansprüche 1 bis 12, **dadurch gekennzeichnet, dass** die Vorrichtung für die Überwachung der Kühlkette von Lebensmitteln, die Überwachung der Feuchtigkeitsbelastung von Produkten, die Qualitätskontrolle von Produkten, die Überwachung der für medizinische Produkte wie Impfstoffe und Medikamente erforderlichen Temperaturen, biomedizinische (Fern-) Messungen oder die Messung und Überwachung von physischen Produktschäden in der Logistik eingesetzt werden kann.

14. System zur Angabe mindestens einer physikalischen Größe und/oder des Zeitablaufs und zu deren elektrischer Übermittlung, wobei das System die Vorrichtung nach einem der Ansprüche 1 bis 12 umfasst, **dadurch gekennzeichnet, dass** eine Lesevorrichtung eingerichtet ist, um drahtlos Daten zu empfangen, die von einem RFID-Tag mit NFC-Technologie oder Bluetooth-Technologie oder einer UHF-Antenne oder einer LF-Antenne gesendet werden.

## Revendications

1. Dispositif permettant d'indiquer au moins une grandeur physique et/ou le passage du temps et de l'envoyer électriquement, dans lequel le dispositif comprend une puce RFID sans batterie (32, 41, 52, 59) et une cellule électrochimique ou un groupe de cellules (42, 43, 45) connecté à l'entrée de la puce RFID, où la décharge ou la charge, ou un changement dans l'état de charge de la dernière cellule de la cellule ou du groupe de cellules (42, 43, 45), provoque l'inversion logique de l'entrée de la puce RFID (32, 41, 52, 59), où le dispositif comporte des données d'identification RFID.

2. Dispositif selon la revendication 1, **caractérisé en ce qu'**une ou plusieurs cellules d'un groupe de cellules ont été connectées à l'entrée de la puce RFID (32, 41, 52, 59), et qu'une modification dans la tension desdites cellules connectées est prévue pour être stockée dans la mémoire de la puce RFID (32, 41, 52, 59).

3. Dispositif selon la revendication 2, **caractérisé en ce que** l'avant-dernière cellule (45) du groupe de cellules (42, 43, 45) est disposée dans l'entrée de la puce RFID (32, 41, 52, 59) pour indiquer que la date de durabilité minimale d'un produit alimentaire ou autre produit est dépassée, et la dernière cellule (42) du groupe de cellules (42, 43, 45) est disposée dans l'entrée de la puce RFID (32, 41, 52, 59) pour indiquer que la date limite de consommation d'un produit alimentaire ou d'un autre produit est dépassée.

4. Dispositif selon l'une quelconque des revendications 1 à 3, **caractérisé en ce que** la décharge d'une cellule souhaitée dans le groupe de cellules (42, 43, 45) provoque l'inversion logique de l'entrée de la puce RFID (32, 41, 52, 59) en question.

5. Dispositif selon l'une quelconque des revendications 1 à 4, **caractérisé en ce que** le dispositif possède une antenne NFC (46), LF, Bluetooth ou UHF pour l'envoi de données.

6. Dispositif selon l'une quelconque des revendications 1 à 5, **caractérisé en ce que** les cellules des groupes de cellules compris dans le dispositif, les résistances ohmiques (R1-R8) et l'antenne (50) sont fabriquées par impression avec une technique rouleau à rouleau, et la résistance NTC (51) et les transistors compris dans le dispositif sont mis en œuvre avec des composants de circuit discrets avec une technique de brasage ou de collage.

7. Dispositif selon l'une quelconque des revendications 1 à 6, **caractérisé en ce que** la tension de sortie de la cellule connectée à l'entrée est fonction d'une ou de plusieurs grandeurs physiques mesurables et du temps de mesure.

8. Dispositif selon la revendication 7, **caractérisé en ce que** ladite grandeur physique est la température, l'humidité relative, le rayonnement électromagnétique et/ou les vibrations.

9. Dispositif selon l'une quelconque des revendications 1 à 8, **caractérisé en ce que** le dispositif est agencé pour décharger ou charger la charge de la cellule avec un composant piézo- ou magnétorésistif.

10. Dispositif selon l'une quelconque des revendications 1 à 9, **caractérisé en ce que** le temps de décharge de la cellule ou du groupe de cellules a été fixé pour être déterminé sur la base de la pression statique ou des impacts à l'aide des matières plastiques électroactives.

11. Dispositif selon l'une quelconque des revendications 1 à 10, **caractérisé en ce que** la première cellule (43) du groupe de cellules est placée à l'extérieur d'une étiquette RFID contenant la puce RFID (32, 41, 52, 59) et que le circuit électrique de ladite première cellule (43) est coupé lorsque le dispositif est mis en service.

12. Dispositif selon l'une quelconque des revendications 1 à 11, **caractérisé en ce que** le circuit connecté à l'entrée de la puce RFID (32, 41, 52, 59) est constitué d'un ordinateur analogique (31) comprenant un transistor ou des transistors.

13. Dispositif selon l'une quelconque des revendications 1 à 12, **caractérisé en ce que** le dispositif est conçu pour être appliqué à la surveillance de la chaîne du froid des produits alimentaires, à la surveillance de l'exposition à l'humidité des produits, au contrôle de la qualité des produits, à la surveillance des températures requises par les produits médicaux tels que les vaccins et les médicaments, aux mesures biomédicales (à distance) ou à la mesure et à la surveillance des dommages physiques des produits en logistique.

14. Système permettant d'indiquer au moins une grandeur physique et/ou le passage du temps et de l'envoyer électriquement, le système comprenant le dispositif selon l'une quelconque des revendications 1 à 12, **caractérisé en ce qu'**un dispositif de lecture est conçu pour recevoir des données sans fil envoyées par une étiquette RFID avec technologie NFC ou Bluetooth ou une antenne UHF ou une antenne LF.
